(19) Europäisches Patentamt / European Patent Office / Office européen des brevets

(11) **EP 3 219 347 B1**

(12) **EUROPEAN PATENT SPECIFICATION**

(45) Date of publication and mention
of the grant of the patent:
**31.10.2018 Bulletin 2018/44**

(51) Int Cl.:
***A61M 5/315*** *(2006.01)*

(21) Application number: **17158019.4**

(22) Date of filing: **27.02.2017**

(54) **GASKET FOR USE IN A SYRINGE**

DICHTUNG ZUR VERWENDUNG IN EINER SPRITZE

JOINT POUR UTILISATION DANS UNE SERINGUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2016 JP 2016051522**

(43) Date of publication of application:
**20.09.2017 Bulletin 2017/38**

(73) Proprietor: **Sumitomo Rubber Industries, Ltd.
Kobe-shi, Hyogo-ken (JP)**

(72) Inventor: **KANEKO, Hiroyuki
Kobe-shi, Hyogo 651-0072 (JP)**

(74) Representative: **Manitz Finsterwald Patentanwälte
PartmbB
Martin-Greif-Strasse 1
80336 München (DE)**

(56) References cited:
**WO-A1-2015/118958      WO-A2-2015/054282
JP-A- 2004 162 761      JP-B2- 3 296 025**

**Description**

[TECHNICAL FIELD]

The present invention relates to a medical syringe, particularly, to a gasket for use in the medical syringe.

[BACKGROUND ART]

Syringes prefilled with a liquid drug (generally referred to as "prefilled syringes") are used as medical syringes. Advantageously, the prefilled syringes permit easy handling without the need for transferring a liquid drug into the syringe, and prevent transfer of a wrong liquid drug into the syringe. For this reason, the prefilled syringes are increasingly used in recent years.

Unlike conventional syringes (into which a liquid drug sucked up from a vial or other container is transferred immediately before use), such a prefilled syringe is required to serve as a container which is kept in contact with the liquid drug for a long period of time.

The syringe typically includes a syringe barrel, a plunger reciprocally movable in the syringe barrel, and a gasket provided at a distal end of the plunger.

The gasket to be used for the syringe is generally made of a crosslinked rubber. It is known that the crosslinked rubber typically contains various crosslinking components, and these crosslinking components and their thermally decomposed products are liable to migrate into the liquid drug when the liquid drug is kept in contact with the gasket. It is also known that these migrating components adversely influence the efficacy and the stability of some liquid drug.

When the syringe is used, the gasket is required to smoothly slide in the syringe barrel. In general, the gasket made of the crosslinked rubber has poorer slidability. Therefore, it is a general practice to apply a silicone oil onto an inner surface of the syringe barrel or a surface of the gasket. However, it is known that the silicone oil adversely influences the efficacy and the stability of some liquid drug.

From this viewpoint, a so-called laminated gasket including a rubber gasket body having a surface laminated with a highly slidable film is often used for the medical syringe. By covering the surface of the rubber gasket body with the highly slidable film, the components of the crosslinked rubber are prevented from migrating into the liquid drug. Further, sufficient slidability is ensured without the use of the silicone oil.

WO 2015/118958 A1 discloses a medical syringe according to the preamble of claim 1.

In JP 3 296025 B2 a syringe is disclosed which comprises a syringe barrel, a gasket and a plunger, wherein the plunger is connected to the gasket via a distal end having a thread engaging a threaded hole provided in the gasket.

A convertible plunger is disclosed in WO 2015/054282 A2 which comprises an internal portion and a gernarally cylindrical exterior surface.

JP 2004 162761 A discloses a further gasket to slide in an outer cylinder.

[CITATION LIST]

[PATENT DOCUMENT]

[Patent Document 1] JP-HEI7(1995)-25953-U

[SUMMARY OF THE INVENTION]

[PROBLEM TO BE SOLVED BY THE INVENTION]

However, the film (mainly, an inert film) to be used for the lamination of the surface of the rubber gasket body of the laminated gasket has no elasticity and, therefore, disadvantageously impairs the elasticity of the inside crosslinked rubber. That is, the inert film has no shape conformability as compared with the rubber. Therefore, when the laminated gasket is inserted into the syringe barrel, the inert film is not brought into intimate contact with the inner wall of the syringe barrel. This may result in leakage of the inside liquid drug, if the liquid drug is highly permeable.

To cope with this problem, a silicone oil is applied to the surface of the inert film, or the diameter of the gasket is increased. In the former case, silicone released in the liquid drug may be agglomerated depending on the type of the liquid drug. In the latter case, resistance (sliding friction) occurring when the gasket is inserted into the syringe barrel is liable to be increased. Therefore, the gasket is liable to have an increased press-insertion resistance or liable to be tilted when the syringe is used.

It is a principal object of the present invention to provide a novel laminated gasket free from the aforementioned problems.

The object of the present invention is further to provide a medical syringe employing the laminated gasket.

[SOLUTION TO PROBLEM]

The problem is solved by the medical syringe according to claim 1. In the laminated gasket, an outer surface of an elastic gasket body such as of a rubber or a thermoplastic elastomer is covered with a plastically deformable resin. Therefore, the laminated gasket tends to suffer from deterioration in gas-tightness at a slidable portion thereof in contact with an inner surface of a glass or resin syringe barrel as compared with the conventional rubber gasket.

If an attempt is made to improve the gas-tightness by increasing the diameter of the gasket, the resistance (sliding friction) occurring when the gasket is inserted into the syringe barrel is increased. Therefore, the gasket is liable to have an increased press-insertion resistance when the syringe is used.

In the present invention, a threaded hole of the gasket is configured so that an innermost thread portion thereof has a slightly smaller thread diameter than the other thread portion thereof. Thus, when a thread of the plunger is in threaded engagement with the innermost thread portion of the threaded hole of the gasket, the smaller thread diameter portion of the gasket body is pressed outward and, therefore, the lamination film is more strongly pressed against the inner wall of the syringe barrel, thereby improving the gas-tightness.

An inventive medical syringe is defined by claims 1 to 7.

According to an inventive aspect of claim 1, more specifically, the laminated gasket for use in the medical syringe includes a main body made of an elastic material, and a film provided on a surface of the main body. The gasket has a circumferential surface portion to be kept in contact with an inner peripheral surface of a syringe barrel of the syringe, a liquid contact surface connected to one of opposite edges of the circumferential surface portion, a bottom surface provided on an other edge side of the circumferential surface portion, and a threaded hole extending from a center of the bottom surface into the main body and adapted to be in threaded engagement with a distal end portion of a plunger of the syringe. The threaded hole has an internal thread provided on an inner peripheral surface thereof as extending from a mouth thereof toward an innermost portion thereof and including a plurality of periodic thread portions. Provided that at least an innermost one of the periodic thread portions of the internal thread adjacent to the innermost portion of the threaded hole has a diameter F1, that the other periodic thread portions of the internal thread each have a diameter F2 and that at least a distal one of periodic thread portions of an external thread provided on the distal end portion of the plunger has a diameter D1, a relationship F2 > D1 > F1 is satisfied.

According to an inventive aspect of claim 2, the diameter F1 of the innermost periodic internal thread portion includes a thread root diameter rt of at least the innermost periodic internal thread portion; the diameter F2 of the other periodic internal thread portions includes a thread root diameter rb of the other periodic internal thread portions; the diameter D1 of the distal periodic external thread portion of the plunger includes a thread peak diameter Dv of at least the distal periodic external thread portion; and rt, rb and Dv satisfy a relationship rb > Dv > rt in the laminated gasket according to claim 1.

According to an inventive aspect of claim 3, the diameter F1 of the innermost periodic internal thread portion includes a thread peak diameter lt of at least the innermost periodic internal thread portion; the diameter F2 of the other periodic internal thread portions includes a thread peak diameter lb of the other periodic internal thread portions; the diameter D1 of the distal periodic external thread portion of the plunger includes a thread root diameter Dm of at least the distal periodic external thread portion; and lt, lb and Dm satisfy a relationship lb > Dm > lt in the laminated gasket according to claim 1.

According to an inventive aspect of claim 4, provided that a distance between the bottom surface and the other edge of the circumferential surface portion is L1 and a distance between the bottom surface and the innermost portion of the threaded hole is L2 as measured axially of the gasket perpendicularly to the bottom surface, L1 and L2 satisfy a relationship $L2 - L1 \geq -1$ mm in the laminated gasket according to claim 1.

According to an inventive aspect of claim 5, the diameter F1 of the internal thread and the diameter D1 of the external thread satisfy a relationship $10\ \mu m \leq D1 - F1 \leq 500\ \mu m$ in the laminated gasket according to claim 1.

According to an inventive aspect of claim 6, the thread root diameter rt and the thread peak diameter Dv satisfy a relationship $10\ \mu m \leq Dv - rt \leq 500\ \mu m$ in the laminated gasket according to claim 2.

According to an inventive aspect of claim 7, the thread peak diameter lt and the thread root diameter Dm satisfy a relationship $10\ \mu m \leq Dm - lt \leq 500\ \mu m$ in the laminated gasket according to claim 3.

According to the invention as defined in claim 1 the medical syringe includes: a tubular syringe barrel; a gasket fitted in the syringe barrel to seal the syringe barrel, a gasket being a laminated gasket and a plunger having a distal end portion kept in threaded engagement with the threaded hole of the laminated gasket, and reciprocally movable in the syringe barrel.

A use method of the medical syringe includes the steps of: when the medical syringe is filled with a liquid drug and stored as a prefilled syringe, bringing the distal end portion of the plunger into threaded engagement with the threaded hole of the laminated gasket to the innermost portion of the threaded hole so that the circumferential surface portion of the laminated gasket is radially pressed against the inner peripheral surface of the syringe barrel; and, when the prefilled syringe is used, bringing the distal end portion of the plunger slightly out of threaded engagement with the threaded hole of the laminated gasket by at least one thread pitch so that the innermost periodic internal thread portion having the diameter F1 is disengaged from the distal periodic external thread portion having the diameter D1 and, in this state, moving the laminated gasket in the syringe barrel by operating the plunger.

[EFFECTS OF THE INVENTION]

According to the present invention, the prefilled syringe and the gasket for the syringe are provided, which are free from leakage of a liquid drug contained in the prefilled syringe.

[BRIEF DESCRIPTION OF THE DRAWINGS]

FIG. 1 is a diagram illustrating a medical syringe according to one embodiment of the present invention in an exploded state.

FIG. 2 is a diagram for explaining a configurational relationship between a gasket and a plunger head 18 according to the embodiment of the present invention.

FIG. 3 is a schematic diagram illustrating an exemplary mold for molding the gasket.

[EMBODIMENTS OF THE INVENTION]

With reference to the attached drawings, one embodiment of the present invention will hereinafter be described specifically.

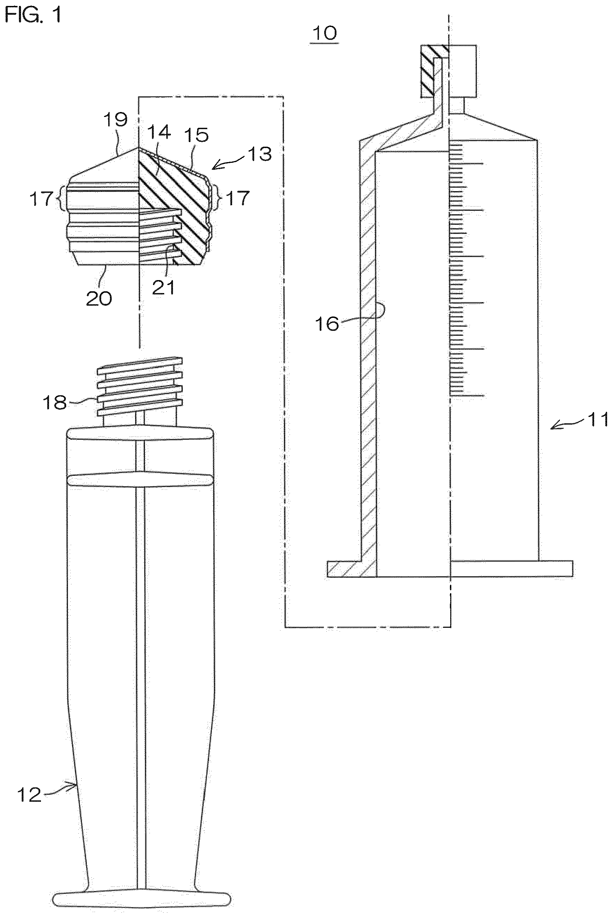

FIG. 1 is a diagram illustrating a medical syringe (so-called prefilled syringe) according to one embodiment of the present invention in an exploded state. In FIG. 1, a syringe barrel 11 and a gasket 13 are shown half in section.

Referring to FIG. 1, the prefilled syringe 10 includes a hollow cylindrical syringe barrel 11, a plunger 12 combined with the syringe barrel 11 and reciprocally movable in the syringe barrel 11, and a gasket 13 attached to a distal end of the plunger 12. The gasket 13 is a so-called laminated gasket which includes a main body 14 made of an elastic material (a rubber, an elastomer or the like), and an inert film 15 provided on a surface of the main body 14. The gasket 13 has a circumferential surface portion 17 which is kept in gas-tight and liquid-tight contact with an inner peripheral surface 16 of the syringe barrel 11.

The plunger 12 includes a resin plate piece, for example, having a cross shape as seen in section, and a head 18 provided at a distal end of the resin plate piece and fitted with the gasket 13. The head 18 is an integral part of the plunger 12 made of a resin and formed with an external thread.

The gasket 13 has a generally cylindrical shape having a short axis. The gasket 13 has a distal end face 19, for example, having a conical center portion projecting at an obtuse angle to serve as a liquid contact surface which is in contact with a liquid drug contained in the syringe barrel 11. The gasket 13 further has a rear end face defined as a bottom surface 20. The gasket 13 has a threaded hole 21 axially recessed as extending from a center of the bottom surface 20 into the main body 14 and formed with an internal thread. The head 18 of the plunger 12 is brought into threaded engagement with the threaded hole 21 of the gasket 13, whereby the gasket 13 is attached to the distal end of the plunger 12.

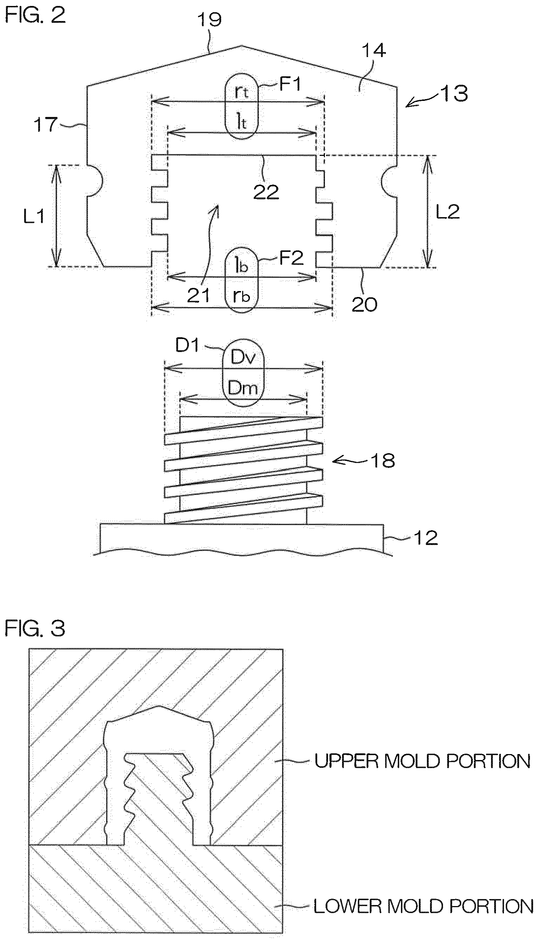

FIG. 2 is a diagram for explaining a configurational relationship between the threaded hole 21 of the gasket 13 and the head 18 of the plunger 12 shown in FIG. 1.

In FIG. 2, the gasket 13 is illustrated in a center vertical section, and the inert film 15 provided on the outer surface of the main body 14 is not shown. The vertical section is suitable for explaining the configuration and the dimensions of the threaded hole 21. The configuration and the dimensions of the external thread of the head 18 of the plunger 12 are also shown.

Referring to FIG. 2, the gasket 13 has the threaded hole 21 extending from the center of the bottom surface 20 thereof into the main body 14 thereof. The threaded hole 21 has an inner peripheral surface formed with the internal thread, which extends from a mouth to an innermost portion 22 of the threaded hole 21 and includes a plurality of periodic thread portions (four periodic thread portions in FIG. 2). An innermost one of the periodic thread portions of the internal thread adjacent to the innermost portion 22 of the threaded hole 21 has a diameter F1, and the other three periodic thread portions of the internal thread adjacent to the mouth of the threaded hole 21 each have a diameter F2.

The diameter F1 of the innermost periodic internal thread portion adjacent to the innermost portion 22 includes the thread root diameter rt and the thread peak diameter lt of the innermost periodic internal thread portion. The diameter F2 of the other three periodic internal thread portions adjacent to the mouth of the threaded hole 21 includes the thread root diameter rb and the thread peak diameter lb of the other three periodic internal thread portions.

On the other hand, the head 18 of the plunger 12 is formed with the external thread. The external thread includes, for example, four periodic thread portions each having a diameter D1. The diameter D1 of the periodic external thread portions includes the thread root diameter Dm and the thread peak diameter Dv of the periodic external thread portions.

F1, F2 and D1 satisfy the following dimensional relationship:

$$F2 > D1 > F1$$

Therefore, rb > Dv > rt and/or lb > Dm > lt.

With this dimensional relationship, when the distal periodic external thread portion of the head 18 of the plunger 12 is brought into threaded engagement with the innermost periodic internal thread portion of the threaded hole 21, a force occurs to press the main body 14 of the gasket 13 radially from the threaded hole 21 toward the circumferential surface portion 17, thereby suppressing the leakage of the liquid drug from between the circumferential surface portion 17 and the inner peripheral surface 16 of the syringe barrel 11.

When the distal periodic external thread portion of the head 18 of the plunger 12 is brought out of the innermost periodic internal thread portion of the threaded hole 21, the circumferential surface portion 17 of the gasket 13 is not radially pressed. Therefore, the gasket 13 can be smoothly slid in the syringe barrel 11 by operating the plunger 12.

A configurational feature of the threaded hole 21 of the gasket 13 is as follows. Provided that a distance between the bottom surface 20 and the innermost portion 22 of the threaded hole 21 of the gasket 13 is L2, that a distance between a lower edge (lower end) of the circumferential surface portion 17 opposite from the liquid contact surface 19 and the bottom surface 20 of the gasket 13 is L1, and that a distance between an upper edge (upper end) of the circumferential surface portion 17 adjacent to the liquid contact surface 19 and the lower edge (lower end) of the circumferential surface portion 17 is L3, the following relationship is satisfied:

$$L3 \geq L2 - L1 \geq -1 \ mm$$

With this relationship, when the innermost periodic internal thread portion of the threaded hole 21 having the diameter F1 is brought into threaded engagement with the head 18 of the plunger 12 to be thereby radially expanded, the diameter of the circumferential surface portion 17 of the gasket 13 is slightly increased. Thus, the gasket 13 is pressed against the inner surface 16 of the syringe barrel with an increased press force.

In the aforementioned arrangement, it is particularly desirable that the thread root diameter rt of at least the innermost periodic internal thread portion adjacent to the innermost portion 22 of the threaded hole 21, the thread root diameter rb of the periodic internal thread portion adjacent to the mouth of the threaded hole 21 and the thread peak diameter Dv of the plunger 12 satisfy the following relationship:

$$rb > Dv > rt$$

If the thread root diameter rt of at least the innermost periodic internal thread portion adjacent to the innermost portion 22 of the threaded hole 21 is greater than the thread peak diameter Dv of the plunger 12, it will be impossible to provide the effects of the present invention.

If the thread root diameter rb of the periodic internal thread portion adjacent to the mouth of the threaded hole 21 is smaller than the thread peak diameter Dv of the plunger 12, resistance occurring when the plunger 12 is inserted into the threaded hole 21 of the gasket 13 will be increased to make the gasket 13 unsuitable for practical applications.

A difference between the thread root diameter rt of the innermost periodic internal thread portion of the threaded hole 21 and the thread peak diameter Dv of the plunger 12 is not particularly limited, but is preferably not less than 10 $\mu$m and not greater than 500 $\mu$m, more preferably not less than 30 $\mu$m and not greater than 300 $\mu$m, further preferably not greater than 150 $\mu$m. If the diameter difference is excessively great, insertion resistance occurring when the external thread of the plunger 12 is inserted to the innermost portion 22 in the threaded hole 21 of the gasket 13 will be excessively great. In some case, it will be impossible to insert the external thread of the plunger 12 to the innermost portion 22 in the threaded hole 21 of the gasket 13. If the diameter difference is excessively small, it will be impossible to provide the desired effects.

[Examples and Comparative Examples]

For production of gaskets of Examples and Comparative Examples, the following film, unvulcanized rubber and crosslinking agent were used. The gaskets were each produced as having a gasket configuration indicated by a reference numeral 13 in FIG. 2 by vulcanization molding of the rubber.

Fluororesin film:

PTFE film (VALFLON (registered trade name) available from Nippon Valqua Industries Ltd.)

The film was treated for adhesion by a method disclosed in JP2012-36249A. The film had a thickness of 70 μm.

Unvulcanized rubber:

Halogenated butyl rubber

Crosslinking agent:

2-di-n-butylamino-4,6-dimercapto-s-triazine Zisnet DB (registered trade name) available from Sankyo Kasei Co., Ltd.

A vulcanization temperature of 180°C, a vulcanization period of 8 minutes and a process pressure of 20 MPa were used for the vulcanization molding.

Molds for the vulcanization molding for the production of the gaskets each included an upper mold portion defining the outermost shape of the gasket, and a lower mold portion defining the internal thread configuration of the gasket as shown in FIG. 3. The thread configuration of the lower mold portion was variously changed for different internal thread configurations of the gaskets.

The thread root diameter and the thread peak diameter of the internal thread portion adjacent to the innermost portion of the threaded hole of the gasket were variously changed for the different internal thread configurations of the gaskets. The distance L2 between the bottom surface of the gasket and the innermost portion of the threaded hole of the gasket was constant, and the thread root diameter and the thread peak diameter of the internal thread portion adjacent to the mouth of the threaded hole of the gasket were also constant (as shown in Table 1). The distance L1 between the lower end of the circumferential surface portion 17 and the bottom surface of the gasket was changed as shown in Table 1. The gaskets each had a maximum diameter of 6.60 mm at the upper edge of the circumferential surface portion 17 thereof.

The gaskets of Examples and Comparative Examples thus produced were each subjected to the following test.

<Liquid Drug Sealability Test>

As a liquid to be sealed in prefilled syringes, an aqueous solution was prepared by dissolving a nonionic surface active agent (POLYSORBATE 80) at a concentration of 0.1 wt.% in water. The gaskets were each inserted into a plastic syringe barrel (of a cycloolefin polymer) having a volume of 1 mL and an inner diameter of 6.35 mm. Thereafter, a plunger (of a polypropylene) having a thread peak diameter $Dv$ of 3.50 mm and a thread root diameter $Dm$ of 1.90 mm was inserted to the innermost portion in the threaded hole of the gasket, and the aqueous solution was injected into the syringe barrel. Then, a distal end of the syringe barrel was capped with a rubber cap. The resulting syringe was allowed to stand at 40°C in an oven for one week. Thereafter, the syringe was taken out of the oven, and a sliding portion of the gasket (a portion present between the circumferential surface portion 17 and a lower circumferential surface portion, hereinafter referred to as "recess") was observed at a magnification of 50X by means of a video microscope (DVM5000 available from Leica Microsystems Inc.) to be checked for the presence of the liquid. Where the liquid reached the recess, it was judged that the liquid was leaked from the syringe.

Table 1

| | | Example 1 | Example 2 | Example 3 | Example 4 | Example 5 | Example 6 | Comparative Example 1 | Comparative Example 2 |
|---|---|---|---|---|---|---|---|---|---|
| Diameter (mm) of thread portion adjacent to innermost portion | | | | | | | | | |
| Thread peak diameter | lt | 2.10 | 2.10 | 2.10 | 2.00 | 2.00 | 2.00 | 2.10 | 2.00 |
| Thread root diameter | rt | 3.45 | 3.40 | 3.40 | 3.20 | 3.20 | 3.00 | 3.50 | 2.70 |
| Diameter (mm) of thread portion adjacent to mouth | | | | | | | | | |
| Thread peak diameter | lb | 2.30 | 2.30 | 2.30 | 2.30 | 2.30 | 2.30 | 2.30 | 2.30 |
| Thread root diameter | rb | 4.00 | 4.00 | 4.00 | 4.00 | 4.00 | 4.00 | 4.00 | 4.00 |
| Distance (mm) between gasket bottom surface and threaded hole innermost portion | L2 | 4.20 | 4.20 | 4.20 | 4.20 | 4.20 | 4.20 | 4.20 | 4.20 |
| Distance (mm) between gasket bottom surface and lower end of sliding portion of gasket adjacent to liquid contact portion in contact with barrel inner surface {circumferential surface portion of gasket} | L1 | 4.20 | 4.00 | 4.50 | 4.50 | 3.70 | 4.70 | 4.50 | 4.20 |
| Difference ($\mu$m) between innermost thread root diameter and plunger thread peak diameter | Dv-rt | 50 | 100 | 100 | 300 | 300 | 500 | 0 | 800 |
| Liquid drug sealability test | | | | | | | | | |
| Liquid leakage | | Not observed | Not observed | Not observed | Not observed | Not observed | Not observed | Observed | *1 |
| | | 0/50 | 0/50 | 0/50 | 0/50 | 0/50 | 0/50 | 8/50 | |
| *1: It was impossible to insert the plunger thread into the innermost thread portion of the gasket. | | | | | | | | | |

For the liquid drug sealability test, 50 samples were produced for each syringe, and the expressions "0/50" and "8/50" shown in Table 1 mean that the liquid leakage occurred in none of the 50 samples and in 8 of the 50 samples, respectively.

As apparent from the results shown in Table 1, the gaskets according to the present invention each had drastically improved gas-tightness.

This application corresponds to Japanese Patent Application No. 2016-051522 filed in the Japan Patent Office on March 15, 2016.

**Claims**

1.  A medical syringe (10) comprising:

    a tubular syringe barrel;
    a gasket (13) fitted in the syringe barrel to seal the syringe barrel; and
    a plunger (12) having a distal end portion kept in threaded engagement with a threaded hole (21) of the gasket (13), and reciprocally movable in the syringe barrel,
    wherein the gasket (13) is a laminated gasket (13) for use in a medical syringe (10), comprising:

    a main body (14) made of an elastic material; and
    a film (15) provided on a surface of the main body;
    the gasket (13) having a circumferential surface portion (17) to be kept in contact with an inner peripheral surface (16) of a syringe barrel (11) of the syringe (10), a liquid contact surface (19) connected to one of opposite edges of the circumferential surface portion (17), a bottom surface (20) provided on an other edge side of the circumferential surface portion (17), and a threaded hole (21) extending from a center of the bottom surface (20) into the main body and adapted to be in threaded engagement with a distal end portion of a plunger of the syringe;
    wherein the threaded hole (21) has an internal thread provided on an inner peripheral surface thereof as extending from a mouth thereof toward an innermost portion thereof and including a plurality of periodic thread portions;
    **characterized in**, provided that at least an innermost one of the periodic thread portions of the internal thread adjacent to the innermost portion of the threaded hole has a diameter F1, that the other periodic thread portions of the internal thread each have a diameter F2 and that at least a distal one of periodic thread portions of an external thread provided on the distal end portion of the plunger has a diameter D1, a relationship F2 > D1 > F1 is satisfied,
    wherein for improving the gas-tightness when a thread of the plunger is in threaded engagement with the innermost thread portion of the threaded hole of the gasket, the smaller thread diameter portion of the gasket body is pressed outward and, therefore, the lamination film is more strongly pressed against the inner wall of the syringe barrel.

2.  The medical syringe (10) according to claim 1,
    **characterized in that** in the laminated gasket the diameter F1 of the innermost periodic internal thread portion includes a thread root diameter rt of at least the innermost periodic internal thread portion;
    the diameter F2 of the other periodic internal thread portions includes a thread root diameter rb of the other periodic internal thread portions;
    the diameter D1 of the distal periodic external thread portion of the plunger includes a thread peak diameter Dv of at least the distal periodic external thread portion; and
    rt, rb and Dv satisfy a relationship rb > Dv > rt.

3.  The medical syringe (10) according to claim 1,
    **characterized in that** in the laminated gasket the diameter F1 of the innermost periodic internal thread portion includes a thread peak diameter lt of at least the innermost periodic internal thread portion;
    the diameter F2 of the other periodic internal thread portions includes a thread peak diameter lb of the other periodic internal thread portions;
    the diameter D1 of the distal periodic external thread portion of the plunger includes a thread root diameter Dm of at least the distal periodic external thread portion; and
    lt, lb and Dm satisfy a relationship lb > Dm > lt.

4. The medical syringe (10) according to claim 1, **characterized in that** in the laminated gasket provided that a distance between the bottom surface (20) and the other edge of the circumferential surface portion is L1 and a distance between the bottom surface (20) and the innermost portion of the threaded hole is L2 as measured axially of the gasket perpendicularly to the bottom surface (20), L1 and L2 satisfy a relationship L2 - L1 $\geq$ -1 mm.

5. The medical syringe (10) according to claim 1, **characterized in that** in the laminated gasket the diameter F1 of the internal thread and the diameter D1 of the external thread satisfy a relationship 10 $\mu$m $\leq$ D1 - F1 $\leq$ 500 $\mu$m.

6. The medical syringe (10) according to claim 2, **characterized in that** in the laminated gasket the thread root diameter rt and the thread peak diameter Dv satisfy a relationship 10 $\mu$m $\leq$ Dv - rt $\leq$ 500 $\mu$m.

7. The medical syringe (10) according to claim 3, **characterized in that** in the laminated gasket the thread peak diameter lt and the thread root diameter Dm satisfy a relationship 10 $\mu$m $\leq$ Dm - lt $\leq$ 500 $\mu$m.

**Patentansprüche**

1. Medizinische Spritze (10), umfassend:

einen rohrförmigen Spritzenhohlzylinder;
eine Dichtung (13), die in den Spritzenhohlzylinder eingesetzt ist, um den Spritzenhohlzylinder abzudichten; und
einen Kolben (12), der einen distalen Endabschnitt aufweist, der in Gewindeeingriff mit einem Gewindeloch (21) der Dichtung (13) gehalten ist und in dem Spritzenhohlzylinder hin- und herbeweglich ist,
wobei die Dichtung (13) eine laminierte Dichtung (13) zur Verwendung in einer medizinischen Spritze (10) ist, umfassend:

einen Hauptkörper (14), der aus einem elastischen Material hergestellt ist; und
einen Film (15), der auf der Oberfläche des Hauptkörpers vorgesehen ist;
wobei die Dichtung (13) einen Umfangsoberflächenabschnitt (17), der in Kontakt mit der Innenumfangsoberfläche (16) eines Spritzenhohlzylinders (11) der Spritze (10) zu halten ist, eine Flüssigkeitskontaktoberfläche (19), die mit einer von entgegengesetzten Kanten des Umfangsoberflächenabschnitts (17) verbunden ist, eine Unterseitenoberfläche (20), die auf einer Seite der anderen Kante des Umfangsoberflächenabschnitts (17) vorgesehen ist, und ein Gewindeloch (21) aufweist, das sich von einer Mitte der Unterseitenoberfläche (20) in den Hauptkörper erstreckt und derart ausgebildet ist, dass es in Gewindeeingriff mit einem distalen Endabschnitt eines Kolbens der Spritze steht;
wobei das Gewindeloch (21) ein Innengewinde aufweist, das an seiner Innenumfangsoberfläche derart vorgesehen ist, dass es sich von seiner Mündung in Richtung seines innersten Abschnitts erstreckt und eine Mehrzahl von periodischen Gewindeabschnitten umfasst;
**dadurch gekennzeichnet, dass**, vorausgesetzt, dass zumindest ein Innerster der periodischen Gewindeabschnitte des Innengewindes benachbart zu dem innersten Abschnitt des Gewindelochs einen Durchmesser F1 aufweist, dass die anderen periodischen Gewindeabschnitte des Innengewindes jeweils einen Durchmesser F2 aufweisen, und dass zumindest ein distaler der periodischen Gewindeabschnitte eines Außengewindes, das an dem distalen Endabschnitt des Kolbens vorgesehen ist, einen Durchmesser D1 aufweist, eine Beziehung F2 > D1 > F1 erfüllt ist,
wobei zur Verbesserung der Gasdichtheit, wenn ein Gewinde des Kolbens in Gewindeeingriff mit dem innersten Gewindeabschnitt des Gewindelochs der Dichtung steht, der kleinere Gewindedurchmesserabschnitt des Dichtungskörpers nach außen gepresst ist und dadurch der Laminierungsfilm stärker gegen die Innenwand des Spritzenhohlzylinders gepresst ist.

2. Medizinische Spritze (10) nach Anspruch 1,

**dadurch gekennzeichnet, dass** in der laminierten Dichtung der Durchmesser F1 des innersten periodischen Innengewindeabschnitts einen Gewindegrunddurchmesser rt von zumindest dem innersten periodischen Innengewindeabschnitt umfasst;
der Durchmesser F2 der anderen periodischen Innengewindeabschnitte einen Gewindegrunddurchmesser rb von den anderen periodischen Innengewindeabschnitten umfasst;
der Durchmesser D1 des distalen periodischen Außengewindeabschnitts des Kolbens einen Gewindespitzendurchmesser Dv von zumindest dem distalen periodischen Außengewindeabschnitt umfasst; und

rt, rb und Dv eine Beziehung rb > Dv > rt erfüllen.

3. Medizinische Spritze (10) nach Anspruch 1,

   **dadurch gekennzeichnet, dass** in der laminierten Dichtung der Durchmesser F1 des innersten periodischen Innengewindeabschnitts einen Gewindespitzendurchmesser It von zumindest dem innersten periodischen Innengewindeabschnitt umfasst;
   der Durchmesser F2 der anderen periodischen Innengewindeabschnitte einen Gewindespitzendurchmesser Ib von den anderen periodischen Innengewindeabschnitten umfasst;
   der Durchmesser D1 des distalen periodischen Außengewindeabschnitts des Kolbens einen Gewindegrunddurchmesser Dm von zumindest dem distalen periodischen Außengewindeabschnitt umfasst; und
   It, Ib und Dm eine Beziehung Ib > Dm > It erfüllen.

4. Medizinische Spritze (10) nach Anspruch 1, **dadurch gekennzeichnet, dass** in der laminierten Dichtung, vorausgesetzt, dass ein Abstand zwischen der Unterseitenoberfläche (20) und der anderen Kante des Umfangsoberflächenabschnitts L1 ist und ein Abstand zwischen der Unterseitenoberfläche (20) und dem innersten Abschnitt des Gewindelochs L2 ist, wie axial der Dichtung senkrecht zu der Unterseitenoberfläche (20) gemessen, L1 und L2 eine Beziehung L2 - L1 $\geq$ -1 mm erfüllen.

5. Medizinische Spritze (10) nach Anspruch 1, **dadurch gekennzeichnet, dass** in der laminierten Dichtung der Durchmesser F1 des Innengewindes und der Durchmesser D1 des Außengewindes eine Beziehung 10 $\mu$m $\leq$ D1 - F1 $\leq$ 500 $\mu$m erfüllen.

6. Medizinische Spritze (10) nach Anspruch 2, **dadurch gekennzeichnet, dass** in der laminierten Dichtung der Gewindegrunddurchmesser rt und der Gewindespitzendurchmesser Dv eine Beziehung 10 $\mu$m $\leq$ Dv - rt $\leq$ 500 $\mu$m erfüllen.

7. Medizinische Spritze (10) nach Anspruch 3, **dadurch gekennzeichnet, dass** in der laminierten Dichtung der Gewindespitzendurchmesser It und der Gewindegrunddurchmesser Dm eine Beziehung 10 $\mu$m $\leq$ Dm - It $\leq$ 500 $\mu$m erfüllen.

**Revendications**

1. Seringue médicale (10) comprenant :

   un fût de seringue tubulaire ;
   un joint (13) logé dans le fût de seringue pour étancher le fût de seringue ; et
   un plongeur (12) ayant une portion d'extrémité distale maintenue en engagement par vissage avec un trou taraudé (21) du joint (13), et déplaçable en va-et-vient dans le fût de seringue,
   dans laquelle le joint (13) est un joint stratifié (13) pour l'utilisation dans une seringue médicale (10), comprenant :

      un corps principal (14) fait d'un matériau élastique ; et
      un film (15) prévu sur une surface du corps principal ;
      le joint (13) ayant une portion de surface circonférentielle (17) destinée à être maintenu en contact avec une surface périphérique intérieur (16) d'un fût de seringue (11) de la seringue (10), une surface de contact pour liquide (19) connectée à l'un des côtés opposés de la portion de surface circonférentielle (17), une surface de fond (20) prévue sur un autre côté de bordure de la portion de surface circonférentielle (17), et un trou taraudé (21) s'étendant depuis un centre de la surface de fond (20) jusque dans le corps principal, et adapté à être en engagement vissé avec une portion d'extrémité distale d'un plongeur de la seringue ;
      dans laquelle le trou taraudé (21) présente un taraudage interne prévu sur une surface périphérique intérieure de celui-ci et s'étendant depuis une embouchure du trou jusqu'à une portion tout à fait intérieure de celui-ci et inclut une pluralité de portions taraudées périodiques ;
      **caractérisée en ce que**, lorsqu'au moins la portion taraudée la plus à l'intérieur parmi les portions taraudées périodiques du taraudage interne, adjacente à la portion la plus intérieure du trou taraudé a pour diamètre F1, que les autres portions taraudées périodiques du taraudage interne ont chacune pour diamètre F2, et au moins une portion distale parmi les portions filetées d'un filetage externe prévu sur la portion d'extrémité distale du plongeur a pour diamètre D1, une relation F2 > D1 > F1 est satisfaite,

dans laquelle, pour améliorer l'étanchéité aux gaz quand un filetage du plongeur est en engagement vissé avec la portion taraudée la plus intérieure du trou taraudé du joint, la portion taraudée de petit diamètre du corps du joint est pressée vers l'extérieur et, par conséquent, le film stratifié est plus fortement pressé contre la paroi intérieure du flux de seringue.

2. Seringue médicale (10) selon la revendication 1,
**caractérisée en ce que**, dans le joint stratifié, le diamètre F1 de la portion taraudée interne périodique la plus à l'intérieur inclut un diamètre de pied de taraudage rt au moins de la portion taraudée interne périodique la plus à l'intérieur ;
le diamètre R2 des autres portions taraudées internes périodiques inclut un diamètre de pied de taraudage rb des autres portions taraudées internes périodiques ;
le diamètre D1 de la portion de filetage externe périodique distale du plongeur inclut un diamètre de sommet de filetage Dv au moins de la portion de filetage externe périodique distale ; et
rt, rb et Dv satisfont la relation rb > Dv > rt.

3. Seringue médicale (10) selon la revendication 1,
**caractérisée en ce que**, dans le joint stratifié, le diamètre F1 de la portion taraudée interne périodique la plus intérieure inclut un diamètre de sommet de taraudage lt au moins de la portion taraudée interne périodique la plus intérieure ;
le diamètre F2 des autres portions taraudées internes périodiques inclut un diamètre de sommet de taraudage lb des autres portions taraudées internes périodiques ;
le diamètre D1 de la portion filetée externe périodique distale du plongeur inclut un diamètre de pied de filetage Dm au moins de la portion filetée externe périodique distale ; et
lt, lb et Dm satisfont la relation lb > Dm > lt.

4. Seringue médicale (10) selon la revendication 1,
**caractérisée en ce que**, dans le joint stratifié, lorsqu'une distance entre la surface de fond (20) et l'autre bordure de la portion de surface circonférentielle est L1 et qu'une distance entre la surface de fond (20) et la portion la plus intérieure du trou taraudé est L2, telle que mesurée axialement du joint et perpendiculairement à la surface de fond (20), L1 et L2 satisfont la relation

$$L2 - L1 \geq -1 \text{ mm}.$$

5. Seringue médicale (10) selon la revendication 1,
**caractérisée en ce que**, dans le joint stratifié, le diamètre F1 du taraudage interne et le diamètre D1 du filetage externe satisfont la relation

$$10 \text{ µm} \leq D1 - F1 \leq 500 \text{ µm}.$$

6. Seringue médicale (10) selon la revendication 2,
**caractérisée en ce que**, dans le joint stratifié, le diamètre de pied de taraudage rt et le diamètre au sommet du taraudage Dv satisfont la relation

$$10 \text{ µm} \leq Dv - rt \leq 500 \text{ µm}.$$

7. Seringue médicale (10) selon la revendication 3,
**caractérisé en ce que** dans le joint stratifié, le diamètre au sommet du taraudage lt et le diamètre au pied du taraudage Dm satisfont la relation

$$10 \text{ µm} \leq Dm - lt \leq 500 \text{ µm}.$$

FIG. 1

10

19  14  15

13

17  17

20  21

18

16

11

12

FIG. 2

FIG. 3

UPPER MOLD PORTION

LOWER MOLD PORTION

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015118958 A1
- JP 3296025 B
- WO 2015054282 A2
- JP 2004162761 A
- JP HEI71995 A
- JP 25953 U
- JP 2012036249 A
- JP 2016051522 A